# NEUE EUROPÄISCHE PATENTSCHRIFT

(11) **EP 0 350 691 B2**
(45) Veröffentlichungstag und Bekanntmachung des Hinweises auf die Entscheidung über den Einspruch: **28.07.1999**
(45) Hinweis auf die Patenterteilung: 24.05.1995
(21) Anmeldenummer: 89111540.4
(22) Anmeldetag: 24.06.1989
(51) Int. Cl.: C07D 213/80, A01N 43/40, A01N 43/54, A01N 43/42, C07D 213/64, C07D 213/79, C07D 213/85, C07D 213/68, C07D 215/22

(54) **Heterocyclisch substituierte alfa-Aryl-acrylsäureester und Fungizide, die diese Verbindungen enthalten**
Heterocyclically substituted alpha-aryl-acrylic-acid esters, and fungicides containing these compounds
Esters de l'acide alpha-aryl-acrylique substitués par un hétérocycle et fongicides contenant ces composés

(30) Priorität: 15.07.1988 DE 3823991
(43) Veröffentlichungstag der Anmeldung: 17.01.1990
(73) Patentinhaber: BASF Aktiengesellschaft, 67063 Ludwigshafen (DE)
(72) Erfinder: Schuetz, Franz, Dr., D-6700 Ludwigshafen (DE); Kuekenhoehner, Thomas, Dr., D-6710 Frankenthal (DE); Wild, Jochen, Dr., D-6705 Deidesheim (DE); Sauter, Hubert, Dr., D-6800 Mannheim (DE); Ammermann, Eberhard, Dr., D-6700 Ludwigshafen (DE); Lorenz, Gisela, Dr., D-6730 Neustadt (DE)

(56) Entgegenhaltungen:
- EP-A- 0 242 081
- EP-A- 0 244 077
- EP-A- 0 278 595
- EP-A- 0 299 694
- EP-A- 0 310 954
- DE-A- 3 620 860

## Beschreibung

Die vorliegende Erfindung betrifft wertvolle neue heterocyclisch substituierte α-Aryl-acrylsäureester mit fungizider Wirkung und Fungizide, die diese Verbindungen enthalten.

Es ist bekannt, Acrylsäuremethylester, z.B. den α-(2-Benzyl-oxyphenyl)-β-methoxyacrylsäuremethylester oder den α-(2-(2-Pyridyl)-oxyphenyl)-β-methoxyacrylsäuremethylester oder den α-(2-(6-Methyl-2-pyridinyl)-oxyphenyl)-β-methoxyacrylsäuremethylester oder den α-(2-Phenoxy-methylphenyl)-β-methoxyacrylsäuremethylester (EP-178 826, 242 070, 242 081) als Fungizide zu verwenden. Ihre fungizide Wirkung ist jedoch unbefriedigend. In EP-A 278 595 wurden heterocyclisch substituierte α-Aryl-acrylsäureester als Fungizide beschrieben.

Es wurde nun gefunden, daß heterocyclisch substituierte α-Aryl-acrylester der allgemeinen Formel I 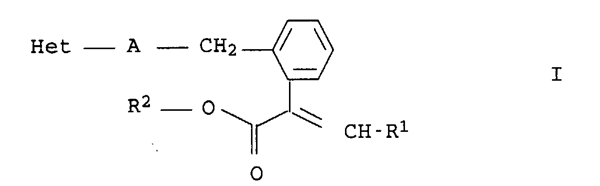 in der
- R¹: C₁-C₄-Alkoxy bedeutet,
- R²: C₁-C₄-Alkyl bedeutet,
- A: für Sauerstoff steht und
- Het: 2-Pyridon-1-yl, 4-Pyridon-1-yl, 3-Chinolyl, 4-Chinolyl, 8-Chinolyl, 2-Pyrimidinon-1-yl, 4-Pyrimidinon-1-yl oder 6-Pyrimidinon-1-yl bedeutet, wobei das heterocyclische Ringsystem gegebenenfalls substituiert ist durch Halogen, C₁-C₈-Alkyl, C₃-C₆-Cycloalkyl, C₁-C₂-Halogenalkyl, C₁-C₄-Alkoxy, C₁-C₄-Alkylthio, C₁-C₄-Alkoxy-C₁-C₄-alkyl, Aryl, Aryl-C₁-C₄-alkyl, C₁-C₄-Alkylcarbonyl, C₁-C₄-Alkoxycarbonyl, Cyano und
- Het: zusätzlich 2-Chinolinyl bedeutet, wobei das heterocyclische Ringsystem substituiert ist durch Halogen, C₁-C₈-Alkyl, C₃-C₆-Cycloalkyl, C₁-C₂-Halogenalkyl, C₁-C₄-Alkoxy, C₁-C₄-Alkylthio, C₁-C₄-Alkoxy-C₁-C₄-alkyl, Aryl, Aryl-C₁-C₄-alkyl, C₁-C₄-Alkylcarbonyl, C₁-C₄-Alkoxycarbonyl, Cyano und
- Het: zusätzlich 2-Pyrimidinyl bedeutet, wobei das heterocyclische Ringsystem mono-substituiert ist durch Brom, C₃-C₆-Cycloalkyl, C₂-Halogenalkyl, C₂-C₄-Alkoxy, C₁-C₄-Alkylthio, C₁-C₄-Alkoxy-C₁-C₄-alkyl, Aryl-C₁-C₄-alkyl, C₁-C₄-Alkylcarbonyl, C₂-C₄-Alkoxycarbonyl, oder
zwei oder drei der folgenden Reste trägt: Chlor, Brom, C₃-C₆-Cycloalkyl, C₁-C₂-Halogenalkyl, C₁-C₄-Alkoxy, C₁-C₄-Alkylthio, C₁-C₄-Alkoxy-C₁-C₄-alkyl, Aryl, Aryl-C₁-C₄-alkyl, C₁-C₄-Alkylcarbonyl, C₁-C₄-Alkoxycarbonyl und Cyano, wobei die folgenden Restekombinationen: Dichlor, Brom-Chlor, Dibrom, Trichlor, Dichlor-trifluormethyl, Chlor-Trifluormethyl, Brom-Trifluormethyl, Chlor-Cyano, Brom-Cyano, Dicyano, Brom-Methoxy, Chlor-Methoxy, Chlor-Acetoxy und Dimethoxy ausgenommen sind und
- Het: zusätzlich 4-Pyrimidinyl bedeutet, wobei das heterocyclische Ringsystem zwei oder drei der folgenden Reste trägt: Fluor, Brom, C₁-C₈-Alkyl, C₃-C₆-Cycloalkyl, C₁-C₂-Halogenalkyl, C₁-C₄-Alkoxy, C₁-C₄-Alkylthio, C₁-C₄-Alkoxy-C₁-C₄-alkyl, Aryl, Aryl-C₁-C₄-alkyl, C₁-C₄-Alkylcarbonyl, C₁-C₄-Alkylcarbonyl und Cyano, wobei
die Restekombinationen: Difluor, Trifluor, Dibrom, Fluor-Brom, Fluor-Trifluormethyl, Fluor-Cyano, Fluor-Methyl, Fluor-Methoxy, Fluor-Dimethyl, Brom-Cyano, Dicyano, Brom-Methoxy, Dimethyl, Dimethoxy und Methyl-Methoxy ausgenommen sind und
- Het: zusätzlich 5-Pyrimidinyl bedeutet, wobei das heterocyclische Ringsystem substituiert ist durch Chlor, Fluor, C₃-C₆-Cycloalkyl, C₁-C₂-Halogenalkyl, C₂-C₄-Alkoxy, C₁-C₄-Alkylthio, C₁-C₄-Alkoxy-C₁-C₄-alkyl, Aryl, Aryl-C₁-C₄-alkyl, C₁-C₄-Alkylcarbonyl, C₁-C₄-Alkoxycarbonyl, Cyano
sowie ihre pflanzenverträglichen Säureadditionssalze und Metallkomplexverbindungen und die N-Oxide der heterocyclischen Verbindungen,
eine ausgezeichnete fungizide Wirkung besitzen, die besser ist als die der bekannten Acrylsäuremethylester.

Die in der allgemeinen Formel aufgeführten Reste können beispielsweise folgende Bedeutung haben:
- R¹: bedeutet C₁-C₄-Alkoxy, z.B. Methoxy, Ethoxy, n- oder iso-Propoxy, Butoxy.
- R²: bedeutet C₁-C₄-Alkyl, z.B. Methyl, Ethyl, n- oder iso-Propyl, n-, iso-, sec.- oder tert.-Butyl.
- Het: bedeutet 2-Pyridon-1-yl, 4-Pyridon-1-yl, 3-Chinolyl, 4-Chinolyl, 8-Chinolyl, 2-Pyrimidinon-1-yl, 4-Pyrimidinon-1-yl oder 6-Pyrimidinon-1-yl, wobei das heterocyclische Ringsystem gegebenenfalls durch einen bis zu drei der folgenden Reste substituiert sein kann:
Halogen (z.B. Fluor, Chlor, Brom), C₁-C₈-Alkyl (z.B. Methyl, Ethyl, n- oder iso-Propyl, n-, iso-, sec.- oder tert.-Butyl, n-, iso-, sec.-, tert.- oder neo-Pentyl, Hexyl, Heptyl, Octyl), C₃-C₆-Cycloalkyl (z.B. Cyclopropyl, Cyclobutyl, Cyclopentyl, Cyclohexyl), C₁-C₂-Halogenalkyl (z.B. Difluormethyl, Trifluormethyl, Chlormethyl, Dichlormethyl, Trichlormethyl, Pentafluorethyl), C₁-C₄-Alkoxy (z.B. Methoxy, Ethoxy, n- oder iso-Propoxy, n-, iso-, sec.- oder tert.-Butoxy), C₁-C₄-Alkoxy-C₁-C₄-alkyl (z.B. Methoxymethyl, Ethoxymethyl, Methoxyethyl, Propoxymethyl), Aryl (z.B. Phenyl), Aryl-C₁-C₄-alkyl (z.B. Benzyl, 2-Phenylethyl, 3-Phenylpropyl, 4-Phenylbutyl), C₁-C₄-Alkylcarbonyl (z.B. Acetyl, Ethylcarbonyl, Propylcarbonyl, Butylcarbonyl, C₁-C₄-Alkoxycarbonyl (z.B. Methoxycarbonyl, Ethoxycarbonyl, Propoxycarbonyl, Butoxycarbonyl), Cyano, C₁-C₄-Alkylthio (z.B. Methylthio, Ethylthio, Propylthio).
- Het: bedeutet 2-Chinolinyl, wobei das heterocyclische Ringsystem durch einen bis zu drei der folgenden Reste substituiert ist: Halogen (z.B. Fluor, Chlor, Brom), C₁-C₈-Alkyl (z.B. Methyl, Ethyl, n- oder iso-Propyl, n-, iso-, sec.- oder tert.-Butyl, n-, iso-, sec.-, tert.- oder neo-Pentyl, Hexyl, Heptyl, Octyl), C₃-C₆-Cycloalkyl (z.B. Cyclopropyl, Cyclobutyl, Cyclopentyl, Cyclohexyl), C₁-C₂-Halogenalkyl (z.B. Difluormethyl, Trifluormethyl, Chlormethyl, Dichlormethyl, Trichlormethyl, Pentafluorethyl), C₁-C₄-Alkoxy (z.B. Methoxy, Ethoxy, n- oder iso-Propoxy, n-, iso-, sec.- oder tert.-Butoxy), C₁-C₄-Alkoxy-C₁-C₄-alkyl (z.B. Methoxymethyl, Ethoxymethyl, Methoxyethyl, Propoxymethyl), Aryl (z.B. Phenyl), Aryl-C₁-C₄-alkyl (z.B. Benzyl, 2-Phenylethyl, 3-Phenylpropyl, 4-Phenylbutyl), C₁-C₄-Alkylcarbonyl (z.B. Acetyl, Ethylcarbonyl, Propylcarbonyl, Butylcarbonyl, C₁-C₄-Alkoxycarbonyl (z.B. Methoxycarbonyl, Ethoxycarbonyl, Propoxycarbonyl, Butoxycarbonyl), Cyano, C₁-C₄-Alkylthio (z.B. Methylthio, Ethylthio, Propylthio).
- Het: bedeutet zusätzlich 2-Pyrimidinyl, wobei das heterocyclische Ringsystem mono-substituiert ist durch:
Brom, C₃-C₆-Cycloalkyl (z.B. Cyclopropyl, Cyclobutyl, Cyclopentyl, Cyclohexyl), C₂-Halogenalkyl (z.B. Pentafluorethyl), C₂-C₄-Alkoxy (z.B. Ethoxy, n- oder iso-Propoxy, n-, iso-, sec.- oder tert.-Butoxy), C₁-C₄-Alkoxy-C₁-C₄-alkyl (z.B. Methoxymethyl, Ethoxymethyl, Methoxyethyl, Propoxymethyl), Aryl-C₁-C₄-alkyl (z.B. Benzyl, 2-Phenylethyl, 3-Phenylpropyl, 4-Phenylbutyl), C₁-C₄-Alkylcarbonyl (z.B. Acetyl, Ethylcarbonyl, Propylcarbonyl, Butylcarbonyl), C₂-C₄-Alkoxycarbonyl (z.B. Ethoxycarbonyl, Propoxycarbonyl, Butoxycarbonyl), C₁-C₄-Alkylthio (z.B. Methylthio, Ethylthio, Propylthio), oder zwei oder drei der folgenden Reste trägt:
Chlor, Brom, C₃-C₆-Cycloalkyl (z.B. Cyclopropyl, Cyclobutyl, Cyclopentyl, Cyclohexyl), C₁-C₂-Halogenalkyl (z.B. Difluormethyl, Trifluormethyl, Chlormethyl, Dichlormethyl, Trichlormethyl, Pentafluorethyl), C₁-C₄-Alkoxy (z.B. Methoxy, Ethoxy, n- oder iso-Propoxy, n-, iso-, sec.- oder tert.-Butoxy), C₁-C₄-Alkoxy-C₁-C₄-alkyl (z.B. Methoxymethyl, Ethoxymethyl, Methoxyethyl, Propoxymethyl), Aryl (z.B. Phenyl), Aryl-C₁-C₄-alkyl (z.B. Benzyl, 2-Phenylethyl, 3-Phenylpropyl, 4-Phenylbutyl), C₁-C₄-Alkylcarbonyl (z.B. Acetyl, Ethylcarbonyl, Propylcarbonyl, Butylcarbonyl), C₁-C₄-Alkoxycarbonyl (z.B. Methoxycarbonyl, Ethoxycarbonyl, Propoxycarbonyl, Butoxycarbonyl), Cyano, C₁-C₄-Alkylthio (z.B. Methylthio, Ethylthio, Propylthio), wobei die folgenden Restekombinationen: Dichlor, Brom-Chlor, Dibrom, Trichlor, Dichlortrifluormethyl, Chlor-Trifluormethyl, Brom-Trifluormethyl, Chlor-Cyano, Brom-Cyano, Dicyano, Brom-Methoxy, Chlor-Methoxy, Chlor-Acetoxy und Dimethoxy ausgenommen sind und
- Het: bedeutet zusätzlich 4-Pyrimidinyl, wobei das heterocyclische Ringsystem zwei oder drei der folgenden Reste trägt:
Fluor, Brom, C₁-C₈-Alkyl (z.B. Methyl, Ethyl, n- oder iso-Propyl, n-, iso-, sec.- oder tert.-Butyl, n-, iso-, sec.-, tert.- oder neo-Pentyl, Hexyl, Heptyl, Octyl), C₃-C₆-Cycloalkyl (z.B. Cyclopropyl, Cyclobutyl, Cyclopentyl, Cyclohexyl), C₁-C₂-Halogenalkyl (z.B. Difluormethyl, Trifluormethyl, Chlormethyl, Dichlormethyl, Trichlormethyl, Pentafluorethyl), C₁-C₄-Alkoxy (z.B. Methoxy, Ethoxy, n- oder iso-Propoxy, n-, iso-, sec.- oder tert.-Butoxy), C₁-C₄-Alkoxy-C₁-C₄-alkyl (z.B. Methoxymethyl, Ethoxymethyl, Methoxyethyl, Propoxymethyl), Aryl (z.B. Phenyl), Aryl-C₁-C₄-alkyl (z.B. Benzyl, 2-Phenylethyl, 3-Phenylpropyl, 4-Phenylbutyl), C₁-C₄-Alkylcarbonyl (z.B. Acetyl, Ethylcarbonyl, Propylcarbonyl, Butylcarbonyl), C₁-C₄-Alkoxycarbonyl (z.B. Methoxycarbonyl, Ethoxycarbonyl, Propoxycarbonyl, Butoxycarbonyl), Cyano, C₁-C₄-Alkylthio (z.B. Methylthio, Ethylthio, Propylthio), wobei die Restkombinationen: Difluor, Trifluor, Dibrom, Fluor-Brom, Fluor-Trifluormethyl, Fluor-Cyano, Fluor-Methyl, Fluor-Methoxy, Fluor-Dimethyl, Brom-Cyano, Dicyano, Brom-Methoxy, Dimethyl, Dimethoxy und Methyl-Methoxy ausgenommen sind und
- Het: bedeutet zusätzlich 5-Pyrimidinyl, wobei das heterocyclische Ringsystem durch einen bis zu drei der folgenden Reste substituiert ist:
Fluor, Chlor, C₃-C₆-Cycloalkyl (z.B. Cyclopropyl, Cyclobutyl, Cyclopentyl, Cyclohexyl), C₁-C₂-Halogenalkyl (z.B. Difluormethyl, Trifluormethyl, Chlormethyl, Dichlormethyl, Trichlormethyl, Pentafluorethyl), C₁-C₄-Alkoxy (z.B. Methoxy, Ethoxy, n- oder iso-Propoxy, n-, iso-, sec.- oder tert.-Butoxy), C₁-C₄-Alkoxy-C₁-C₄-alkyl (z.B. Methoxymethyl, Ethoxymethyl, Methoxyethyl, Propoxymethyl), Aryl (z.B. Phenyl), Aryl-C₁-C₄-alkyl (z.B. Benzyl, 2-Phenylethyl, 3-Phenylpropyl, 4-Phenylbutyl), C₁-C₄-Alkylcarbonyl (z.B. Acetyl, Ethylcarbonyl, Propylcarbonyl, Butylcarbonyl), C₁-C₄-Alkoxycarbonyl (z.B. Methoxycarbonyl, Ethoxycarbonyl, Propoxycarbonyl, Butoxycarbonyl), Cyano, C₁-C₄-Alkylthio (z.B. Methylthio, Ethylthio, Propylthio).

Die neuen Verbindungen können durch Umsetzung mit Säuren auch in pflanzenverträgliche Säureadditionssalze der anorganischen oder organischen Säuren überführt werden, wie beispielsweise in Salze der Salzsäure, Bromwasserstoffsäure, Salpetersäure, Oxalsäure, Essigsäure, Schwefelsäure, Phosphorsäure oder Dodecylbenzolsulfonsäure. Die Wirksamkeit der Salze geht auf das Kation zurück, so daß die Wahl des Anions im allgemeinen beliebig ist.

Ferner lassen sich die neuen Verbindungen nach bekannten Methoden in Metallkomplexe überführen. Das kann durch Umsetzung dieser Verbindungen mit Metallsalzen, z.B. der Metalle, Kupfer, Zink, Eisen, Mangan oder Nickel, beispielsweise Kupfer(II)-chlorid, Zink(II)-chlorid, Eisen(III)-chlorid, Kupfer(II)-nitrat, Mangan(II)-chlorid oder Nickel(II)-bromid erfolgen.

Durch Umsetzung der neuen Verbindungen mit Oxidationsmitteln z.B. mit m-Chlorperbenzoesäure erhält man die N-Oxide der heterocyclischen Verbindungen.

Die neuen Verbindungen können beispielsweise nach folgenden Verfahren hergestellt werden:

Zur Herstellung der neuen Verbindungen der allgemeinen Formel werden die heterocyclischen Verbindungen der allgemeinen Formel Het-AH (II) mit α-Brommethylphenylacrylestern der allgemeinen Formel III umgesetzt. 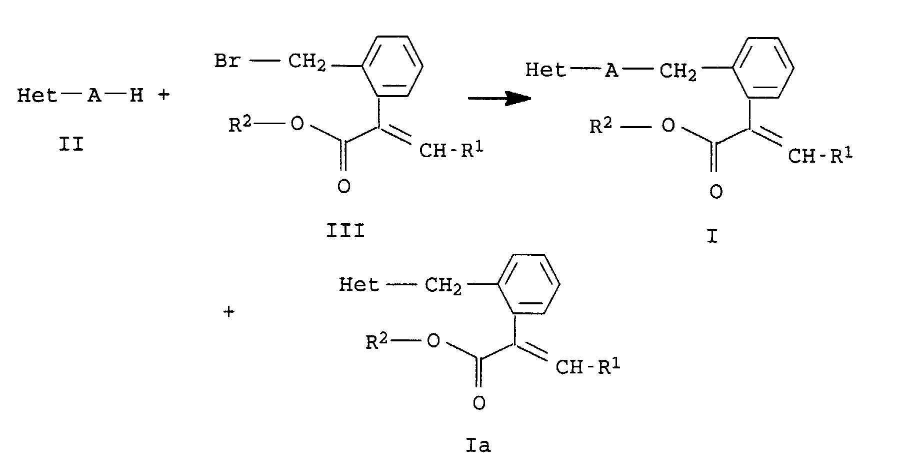 Die neuen Verbindungen der allgemeinen Formel Ia (Het = 2-Pyridon-1-yl, 4-Pyridon-1-yl, 2-Pyrimidinon-1-yl, 4-Pyrimidinon-1-yl, 6-Pyrimidinon-1-yl; R¹ und R² haben die oben angegebene Bedeutung) entstehen bei der Reaktion der heterocyclischen Verbindungen der allgemeinen Formel Het-OH IIa mit α-Brommethylphenyl-acrylestern der allgemeinen Formel III gemäß obiger Reaktionsgleichung.

α-Brommethylphenyl-acrylester der allgemeinen Formel III sind bekannt aus DE-A 35 19 280, DE-A 35 45 318 und DE-A 35 45 319.

Eine Verbindung Het-OH, z.B. das 2-Hydroxypyridin, kann in Form von zwei tautomeren Formen reagieren 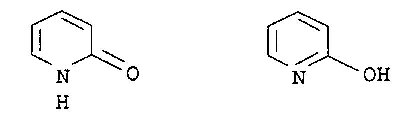 d.h. als 2-Hydroxypyridin oder 2-Pyridon.

Entsprechend findet die Umsetzung mit der Brommethylverbindung am N oder am O, d.h. in 1-Stellung oder in 2-Stellung statt. Entsprechendes gilt beispielsweise für die Verbindung 4-Pyridon. Die Endprodukte, die im heterocyclischen Rest die CO-Gruppe im Ring enthalten, sind über N d.h. in 1-Stellung mit der CH₂-Gruppe des Benzylrestes verbunden.

Die Verbindungen der allgemeinen Formel la sind konstituionsisomer zu den entsprechenden Verbindungen der allgemeinen Formel I. Die Produktverteilung zwischen I und la ist abhängig von den Substituenten am Heterocyclus. Der prozentuale Anteil von Ib an der Gesamtausbeute (I + la) kann zwischen 0 und 100 % betragen.

Die Trennung der Konstitutionsisomeren I und la in die reinen Verbindungen kann z.B. durch fraktionierte Kristallisation oder durch Chromatographie in der üblichen Weise erfolgen.

Die Umsetzungen zu den Verbindungen I und la können z.B. in einem inerten Lösungs- oder Verdünnungsmittel (z.B. Aceton, Acetonitril, Dimethylsulfoxid, Dioxan, Dimethylformamid, N-Methylpyrrolidon, N,N'-Dimethylpropylenharnstoff oder Pyridin) unter Verwendung einer Base (z.B. Natriumcarbonat, Kaliumcarbonat) durchgeführt werden. Außerdem kann es von Vorteil sein, der Reaktionsmischung einen Katalysator wie z.B. Tris-(3,6-dioxoheptyl)-amin zuzusetzen (J. Org. Chem. 50 (1985) 3717).

Alternativ kann auch so vorgegangen werden, daß man die Verbindungen der allgemeinen Formel II zunächst mit einer Base (z.B. Natriumhydroxid oder Kaliumhydroxid) in die entsprechenden Natrium- oder Kaliumsalze überführt und diese dann in einem inerten Lösungs-oder Verdünnungsmittel (z.B. Dimethylformamid) mit den α-Brommethylphenyl-acrylestern III zu den entsprechenden Verbindungen der allgemeinen Formel I bzw.la umsetzt.

Die heterocyclischen Ausgangsverbindungen der allgemeinen Formel III sind entweder bekannt oder können durch Verfahren analog zu bekannten Verfahren hergestellt werden. Entsprechende Herstellverfahren sind z.B. beschrieben in: EP 224 217; DE-25 31 035; J. Heterocyclic Chem. 20 (1983) 219; J. Heterocyclic Chem. 24 (1987) 709.

Die neuen Verbindungen der allgemeinen Formel I können aufgrund ihrer C = C-Doppelbindung sowohl als E- als auch als Z-lsomere vorliegen. Sowohl die einzelnen isomeren Verbindungen als auch ihre Gemische werden von der

Erfindung erfaßt und können als Fungizide verwendet werden. Bevorzugt werden die bei der Synthese anfallenden Gemische als Fungizide verwendet.

Die folgenden Beispiele sollen die Herstellung der neuen Wirkstoffe erläutern.

### Beispiel 1

α-[2-(4'-Methyl-2'-chinolyl)-oxymethylphenyl]-β-methoxy-acrylsäuremethylester (Verbindung Nr. 134) 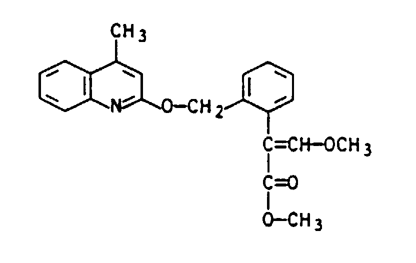 15,9 g (0,1 mol) 4-Methyl-2-hydroxy-chinolin und 4,0 g (0,1 mol) NaOH werden in 150 ml Ethanol gelöst und zwei Stunden bei Raumtemperatur gerührt. Der ausgefallene Niederschlag wird abgesaugt, mit Diethylether gewaschen und in 400 ml Dimethylformamid suspendiert. Anschließend werden 28,5 g (0,1 mol) α-(2-Brommethylphenyl)-β-methoxy-acrylsäuremethylester zugegeben. Man rührt 48 Stunden bei Raumtemperatur, engt das Reaktionsgemisch ein und nimmt den Rückstand in Methylenchlorid auf. Die organische Phase wird mit Wasser gewaschen, über MgSO₄ getrocknet und eingeengt. Das erhaltene Öl wird an Kieselgel (Cyclohexan:Essigester 5:1) chromatographiert. Man erhält 13,1 g (36 %) der Titelverbindung als farbloses Öl.

### Beispiel 2

α-[2-(2',6'-Bis-trifluormethyl-4'-pyrimidinyl)-oxymethyl-phenyl]-β-methoxy-acrylsäuremethylester (Verbindung Nr. 202) 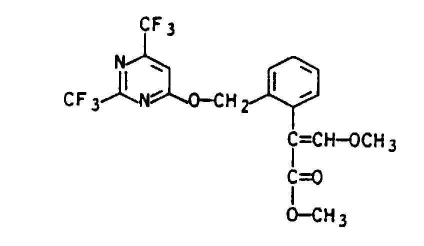 23,2 g (0,1 mol) 2,6-Bis-trifluormethyl-4-hydroxypyrimidin, 28,5 g (0,1 mol) α-(2-Brommethylphenyl)-β-methoxy-acrylsäuremethylester und 20,7 g (0,15 mol) Kaliumcarbonat werden in 250 ml Dimethylformamid 48 Stunden bei Raumtemperatur gerührt. Anschließend wird das Reaktionsgemisch eingeengt, der Rückstand in Methylenchlorid aufgenommen, die organische Phase mit Wasser gewaschen und über MgSO₄ getrocknet. Das nach dem Einengen erhaltene Öl wird über Kieselgel (Cyclohexan) filtriert. Nach dem Entfernen des Lösungsmittels erhält man 24 g (55 %) der Titelverbindung in Form farbloser Kristalle. In entsprechender Weise lassen sich die folgenden Verbindungen herstellen:

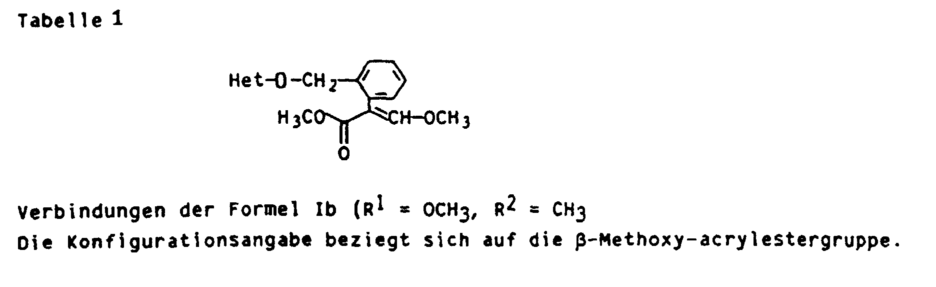

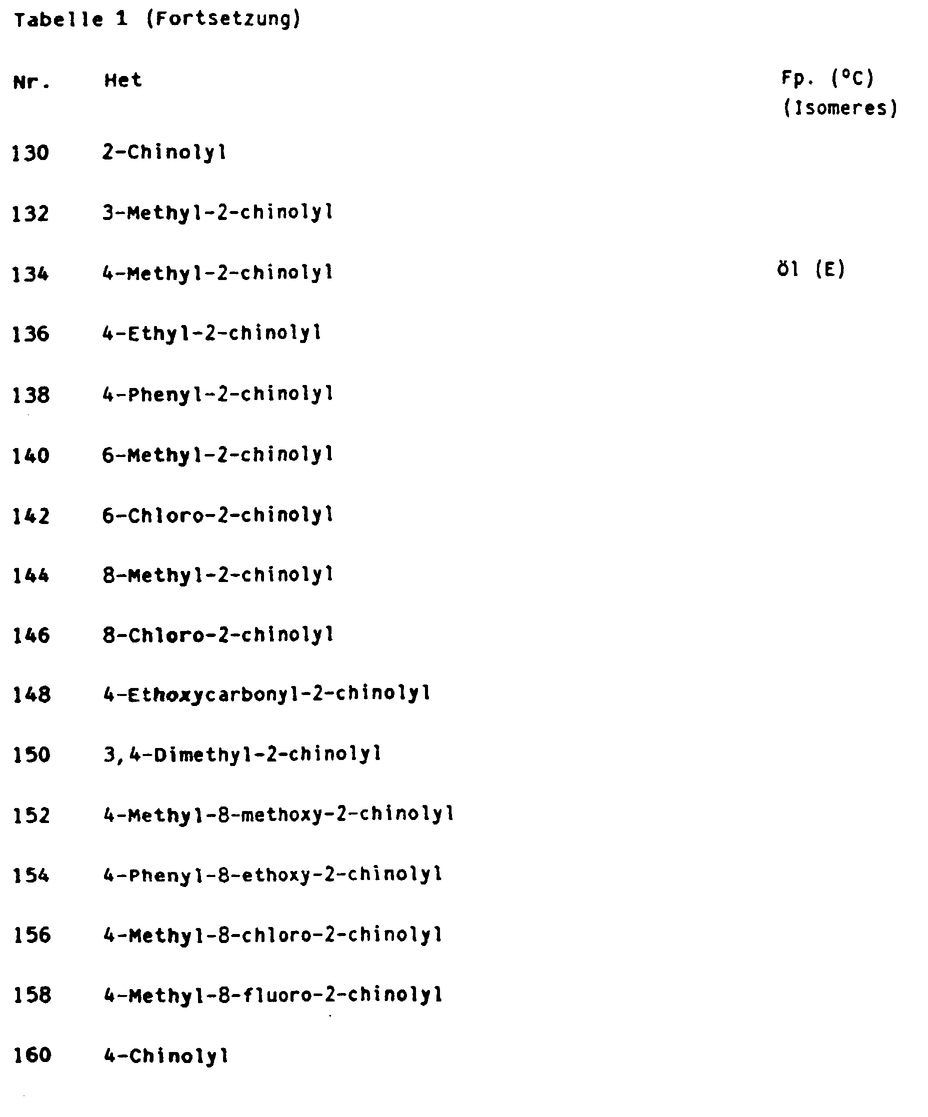

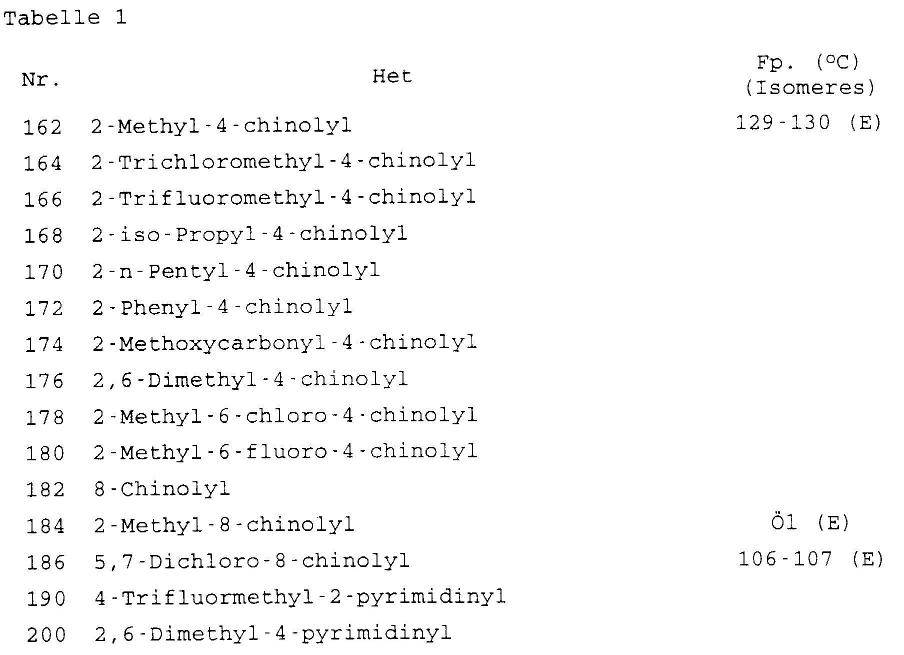

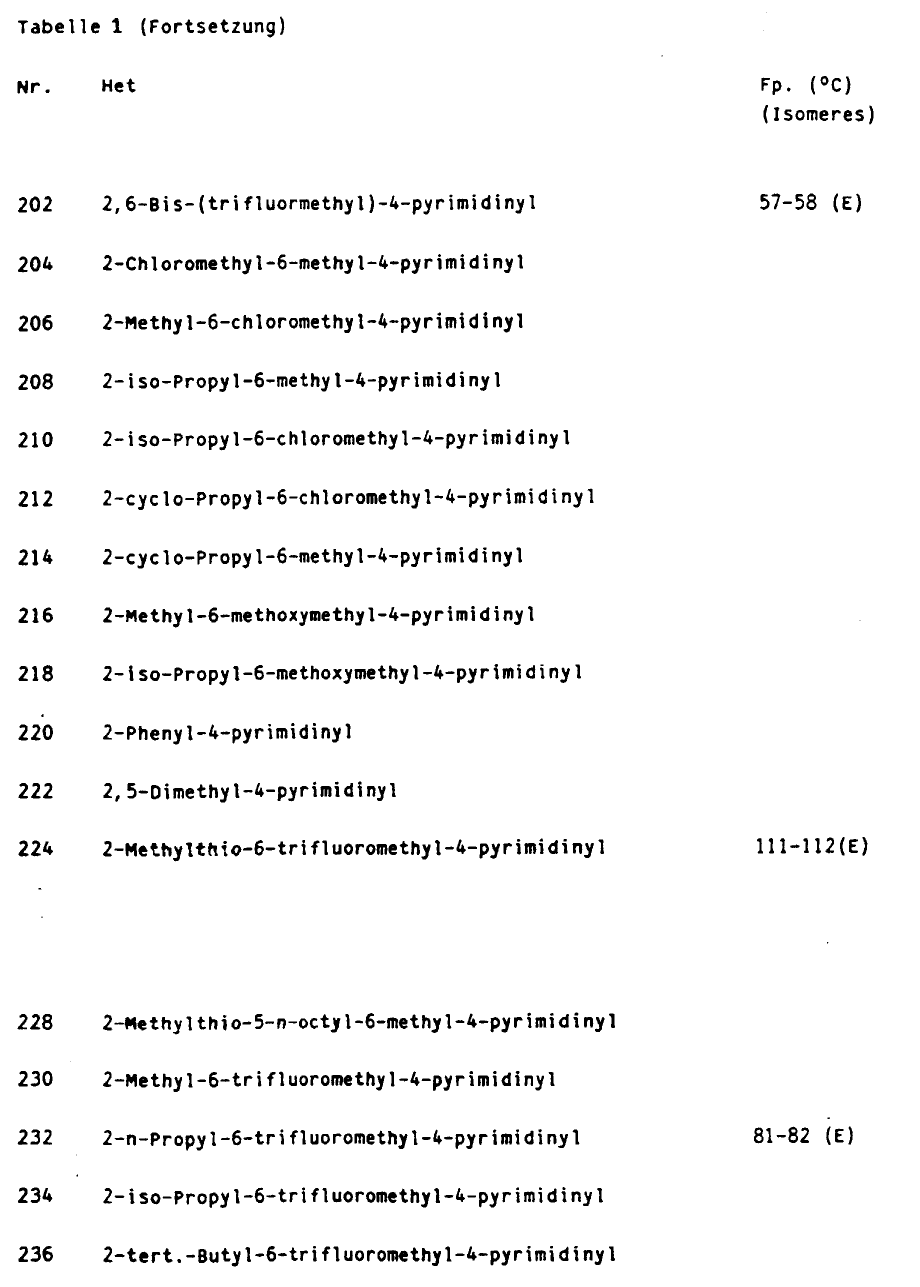

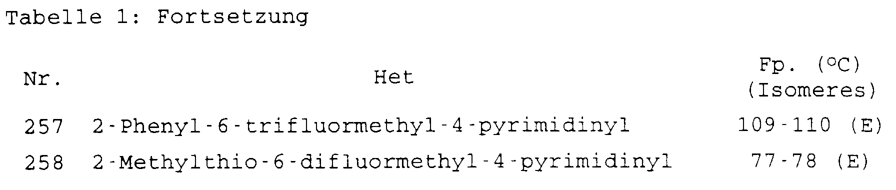

**Tabelle 2**

| NMR-Daten ausgewählter Verbindungen aus der Tabelle 1. Die chemische Verschiebung (δ) wird in ppm relativ zu Tetramethylsilan angegeben. Als Lösungsmittel dient CDCl₃. |
|---|
| Verbindung Nr. 134 |
| 2.60 (s, 3H); 3.68 (s, 3H); 3.79 (s, 3H); 5.43 (s, 2H); 6.75 (s, 1H); 7.13-7.87 (m, 8H); 7.57 (m, 1H). |

| Verbindung Nr. 162 |
|---|
| 2.65 (s, 3H); 3.69 (s, 3H); 3.80 (s, 3H); 5.16 (s, 2H); 6.57 (s, 1H); 7.20-7.67 (m, 6H); 7.57 (s, 1H); 7.93 (s, 1H); 8.19 (s, 1H). |

| Verbindung Nr. 184 |
|---|
| 2.80 (s, 3H); 3.72 (s, 3H); 3.79 (s, 3H); 5.39 (s, 2H); 6.85 (d, 1H); 7.16-7.28 (m, 6H); 7.64 (m, 2H); 7.95 (d, 1H). |

| Verbindung Nr. 186 |
|---|
| 3.68 (s, 3H); 3.79 (s, 3H); 5.42 (s, 2H); 7.16-7.60 (m, 4H); 7.50 (s, 1H); 7.65 (s, 1H); 8.04 (d, 1H); 8.53 (d, 1H); 9.02 (d, 1H). |

| Verbindung Nr. 202 |
|---|
| 3.68 (s, 3H); 3.83 (s, 3H); 5.50 (s, 2H); 7.16 (s, 1H); 7.20-7.58 (m, 4H); 7.60 (s, 1H). |

| Verbindung Nr. 224 |
|---|
| 2.55 (s, 3H); 3.69 (s, 3H); 3.81 (s, 3H); 5.40 (s, 2H); 6.68 (s, 1H); 7.29 -7.54 (m, 4H); 7.60 (s, 1H). |

| Verbindung Nr. 226 |
|---|
| 2.57 (s, 3H); 3.72 (s, 3H); 3.84 (s, 3H); 5.48 (s, 2H); 7.20-7.60 (m, 4H); 7.62 (s, 1H). |

| Verbindung Nr. 232 |
|---|
| 1.01 (t, 3H); 1.87 (sext., 2H); 2.90 (t, 2H); 3.70 (s, 3H); 3.81 (s, 3H); 5.40 (s, 2H); 6.86 (s, 1H); 7.20-7.58 (m, 4H); 7.60 (s, 1H). |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen, Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:
I. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist.
II. 20 Gew.-Teile einer erfindungsgemäßen Verbindung werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.
III. 20 Gew.-Teile einer erfindungsgemäßen Verbindung werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.
IV. 20 Gew.-Teile einer erfindungsgemäßen Verbindung werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.
V. 80 Gew.-Teile einer erfindungsgemäßen Verbindung werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.
VI. 3 Gew.-Teile einer erfindungsgemäßen Verbindung werden mit 97 Gew.-Teilen feinteiligem Kaolin innig vermischt. Man erhält auf diese Weise ein Stäubemittel, das 3 Gew.% des Wirkstoffs enthält.
VII. 30 Gew.-Teile einer erfindungsgemäßen Verbindung werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VIII. 40 Gew.-Teile einer erfindungsgemäßen Verbindung werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.
IX. 20 Gew.-Teile einer erfindungsgemäßen Verbindung werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:
Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithiolo-[4,5-b]-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon, Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-lod-benzoesäure-anilid,
N-Formyl-N-morpholin-2,2,2-trichlorethylacetal,
Piperazin-1,4-diylbis-(1-(2,2,2-trichlor-ethyl)-formamid,
1 -(3,4-Dichloranilino)-1-formylamino-2,2,2-trichlorethan,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin.
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

### Anwendungsbeispiele

Als Vergleichswirkstoffe wurden α-(2-Benzyl-oxyphenyl)-β-methoxy-acrylsäuremethylester (A) - bekannt aus DE 3 519 282 - und α-(2-Phenoxy-methylphenyl)-β-methoxy-acrylsäuremethylester (B) - bekannt aus DE-35 45 319 - und α-(2-(2-Pyridyl)-oxyphenyl-β-methoxyacrylsäuremethylester (D) - bekannt aus EP-178 826 - und α-(2-(6-Methyl-2-pyridyl)-oxyphenyl-β-methoxyacrylsäuremethylester (C) - bekannt aus EP-242 081 benutzt.

### Anwendungsbeispiel 1

### Wirksamkeit gegen Pseudocercosporella herpotrichoides

Weizenpflanzen der Sorte "Frühgold" wurden im Ein-Blatt-Stadium mit wäßrigen Wirkstoffaufbereitungen, die 80 % (Gew.%) Wirkstoff und 20 % Emulgiermittel in der Trockensubstanz enthielten, bis zur Tropfnässe besprüht. 24 Stunden später wurden diese Pflanzen mit einer Sporensuspension von Pseudocercosporella herpotrichoides inokuliert. Zur optimalen Entwicklung der Pflanzenkrankheiten wurden die Pflanzen anschließend für 1 Woche in eine Klimakammer mit 16 bis 18°C und einer rel. Luftfeuchtigkeit >95 % gestellt. Dann wurden die Pflanzen für zwei weitere Wochen im Gewächshaus bei 15 bis 17°C kultiviert. Die Auswertung erfolgte durch Beurteilung der Symptome am unteren Teil des Pflanzenstengels.

Das Ergebnis des Versuches zeigt, daß die Verbindung Nr. 162 bei der Anwendung als 0,1 %ige Sprizbrühe eine bessere fungizide Wirkung zeigt (90 %) als die bekannten Wirkstoffe A und B (70 %).

### Anwendungsbeispiel 2

### Wirksamkeit gegen Rebenperonospora

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst 48 Stunden in einer wasserdampfgesättigten Kammer bei 24°C und anschließend für 5 Tage in einem Gewächshaus mit Temperaturen zwischen 20 und 30°C aufgestellt. Nach dieser Zeit wurden die Pflanzen zur Beschleunigung des Sporangienträgerbruches abermals 16 Stunden in der feuchten Kammer aufgestellt. Dann erfolgte die Beurteilung des Ausmaßes des Pilzausbruches auf den Blattunterseiten.

Das Ergebis des Versuches zeigt, daß die Verbindung 232 bei der Anwendung als 0,006 %ige Spritzbrühe eine bessere fungizide Wirkung zeigt (100 %) als die bekannten Wirkstoffe C und D (70 %).

## Patentansprüche

1. Heterocyclisch substituierte α-Aryl-acrylester der allgemeinen Formel I, 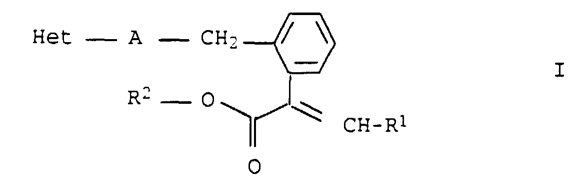 in der
R¹ C₁-C₄-Alkoxy bedeutet,
R² C₁-C₄-Alkyl bedeutet,
A für Sauerstoff steht und
Het 2-Pyridon-1-yl, 4-Pyridon-1-yl, 3-Chinolyl, 4-Chinolyl, 8-Chinolyl, 2-Pyrimidinon-1-yl, 4-Pyrimidinon-1-yl oder 6-Pyrimidinon-1-yl bedeutet, wobei das heterocyclische Ringsystem gegebenenfalls substituiert ist durch Halogen, C₁-C₈-Alkyl, C₃-C₆-Cycloalkyl, C₁-C₂-Halogenalkyl, C₁-C₄-Alkoxy,C₁-C₄-Alkylthio, C₁-C₄-Alkoxy-C₁-C₄-alkyl, Aryl, Aryl-C₁-C₄-akyl, C₁-C₄-Alkylcarbonyl, C₁-C₄-Alkoxycarbonyl, Cyano und
Het zusätzlich 2-Chinolinyl bedeutet, wobei das heterocyclische Ringsystem substituiert ist durch Halogen, C₁-C₈-Alkyl, C₃-C₆-Cycloalkyl, C₁-C₂-Halogenalkyl, C₁-C₄-Alkoxy, C₁-C₄-Alkylthio, C₁-C₄-Alkoxy-C₁-C₄-alkyl, Aryl, Aryl-C₁-C₄-alkyl, C₁-C₄-Alkylcarbonyl, C₁-C₄-Alkoxycarbonyl, Cyano und
Het zusätzlich 2-Pyrimidinyl bedeutet, wobei das heterocyclische Ringsystem mono-substituiert ist durch Brom, C₃-C₆-Cycloalkyl, C₂-Halogenalkyl, C₂-C₄-Alkoxy, C₁-C₄-Alkylthio, C₁-C₄-Alkoxy-C₁-C₄-alkyl, Aryl-C₁-C₄-alkyl, C₁-C₄-Alkylcarbonyl, C₂-C₄-Alkoxycarbonyl, oder
zwei oder drei der folgenden Reste trägt: Chlor, Brom, C₃-C₆-Cycloalkyl, C₁-C₂-Halogenalkyl, C₁-C₄-Alkoxy, C₁-C₄-Alkylthio, C₁-C₄-Alkoxy-C₁-C₄-alkyl, Aryl, Aryl-C₁-C₄-alkyl, C₁-C₄-Alkylcarbonyl, C₁-C₄-Alkoxycarbonyl und Cyano, wobei die folgenden Restekombinationen: Dichlor, Brom-Chlor, Dibrom, Trichlor, Dichlortrifluormethyl, Chlor-Trifluormethyl, Brom-Trifluormethyl, Chlor-Cyano, Brom-Cyano, Dicyano, Brom-Methoxy, Chlor-Methoxy, Chlor-Acetoxy und Dimethoxy ausgenommen sind und
Het zusätzlich 4-Pyrimidinyl bedeutet, wobei das heterocyclische Ringsystem zwei oder drei der folgenden Reste trägt: Fluor, Brom, C₁-C₈-Alkyl, C₃-C₆-Cycloalkyl, C₁-C₂-Halogenalkyl, C₁-C₄-Alkoxy, C₁-C₄-Alkylthio, C₁-C₄-Alkoxy-C₁-C₄-alkyl, Aryl, Aryl-C₁-C₄-alkyl, C₁-C₄-Alkylcarbonyl, C₁-C₄-Alkoxycarbonyl, und Cyano, wobei
die Restekombinationen: Difluor, Trifluor, Dibrom, Fluor-Brom, Fluor-Trifluormethyl, Fluor-Cyano, Fluor-Methyl, Fluor-Methoxy, Fluor-Dimethyl, Brom-Cyano, Dicyano, Brom-Methoxy, Dimethyl, Dimethoxy und Methyl-Methoxy ausgenommen sind und
Het zusätzlich 5-Pyrimidinyl bedeutet, wobei das heterocyclische Ringsystem substituiert ist durch Chlor, Fluor, C₃-C₆-Cycloalkyl, C₁-C₂-Halogenalkyl, C₂-C₄-Alkoxy, C₁-C₄-Alkylthio, C₁-C₄-Alkoxy-C₁-C₄-alkyl, Aryl, Aryl-C₁-C₄-alkyl, C₁-C₄-Alkylcarbonyl, C₁-C₄-Alkoxycarbonyl, Cyano
sowie ihre pflanzenverträglichen Säureadditionssalze und Metallkomplexverbindungen und die N-Oxide der heterocyclischen Verbindungen.

2. Verfahren zur Herstellung von heterocyclisch substituierten α-Arylacrylestern der Formel I gemäß Anspruch 1, dadurch gekennzeichnet, daß man eine heterocyclische Verbindung der allgemeinen Formel II Het-(A)ₙ-H mit einem α-(2-Brommethylphenyl)-acrylester der allgemeinen Formel III 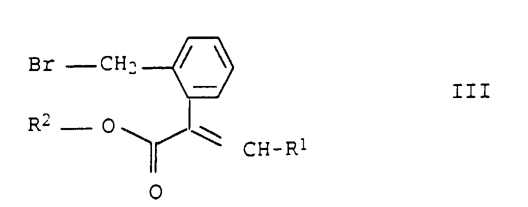 in einem Verdünnungsmittel in Gegenwart einer Base und gegebenenfalls in Gegenwart eines Katalysators umsetzt.

3. Verfahren zur Bekämpfung von Pilzen, dadurch gekennzeichnet, daß man die Pilze oder die von Pilzbefall bedrohten Materialien, Pflanzen, Saatgüter oder den Erdboden mit einer fungizid wirksamen Menge eines heterocyclisch substituierten α-Aryl-acrylesters der allgemeinen Formel I gemäß Anspruch 1 oder mit dessen pflanzenverträglichem Säureadditionssalz oder Metallkomplexverbindung oder dem N-Oxid der heterocyclischen Verbindung behandelt.

4. Fungizid, enthaltend einen inerten Trägerstoff und eine fungizid wirksame Menge eines heterocyclisch substituierten α-Aryl-acrylesters der allgemeinen Formel I gemäß Anspruch 1 oder dessen pflanzenverträglichem Säureadditionssalz oder Metallkomplexverbindung oder dem N-Oxid der heterocyclischen Verbindung.

5. Verbindung der Formel I gemäß Anspruch 1, dadurch gekennzeichnet, daß R¹ Methoxy und R² Methyl und Het 2-Methyl-4-chinolyl bedeutet.

## Claims

1. An α-arylacrylate substituted by a heterocyclic radical, of the general formula I 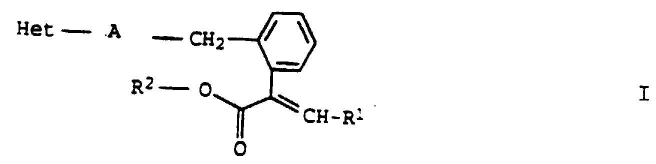
where R¹ is C₁-C₄-alkoxy, R² is C₁-C₄-alkyl, A is oxygen and Het is 4-pyridon-1-yl, 4-pyridon-1-yl, 3-quinolyl, 4-quinolyl, 8-quinolyl, 2-pyrimidinon-1-yl, 4-pyrimidinon-1-yl or 6-pyrimidinon-1-yl, where the heterocyclic ring system is unsubstituted or substituted by halogen, C₁-C₈-alkyl, C₃-C₆-cycloalkyl, C₁-C₂-haloalkyl, C₁-C₄-alkoxy, C₁-C₄-alkylthio, C₁-C₄-alkoxy-C₁-C₄-alkyl, aryl, aryl-C₁-C₄-alkyl, C₁-C₄-alkylcarbonyl, C₁-C₄-alkoxycarbonyl or cyano, and
Het is additionally 2-quinolyl, where the heterocyclic ring system is substituted by halogen, C₁-C₈-alkyl, C₃-C₆-cycloalkyl, C₁-C₂-haloalkyl, C₁-C₄-alkoxy, C₁-C₄-alkylthio, C₁-C₄-alkoxy-C₁-C₄-alkyl, aryl, aryl-C₁-C₄-alkyl, C₁-C₄-alkylcarbonyl, C₁-C₄-alkoxycarbonyl or cyano, and
Het is additionally 2-pyrimidinyl, where the heterocyclic ring system is monosubstituted by bromine, C₃-C₆-cycloalkyl, C₂-haloalkyl, C₂-C₄-alkoxy, C₁-C₄-alkylthio, C₁-C₄-alkoxy-C₁-C₄-alkyl, aryl-C₁-C₄-alkyl, C₁-C₄-alkylcarbonyl or C₂-C₄-alkoxycarbonyl, or carries two or three of the following radicals: chlorine, bromine, C₃-C₆-cycloalkyl, C₁-C₂-haloalkyl, C₁-C₄-alkoxy,C₁-C₄-alkylthio, C₁-C₄-alkoxy-C₁-C₄-alkyl, aryl, aryl-C₁-C₄-alkyl,C₁-C₄-alkylcarbonyl, C₁-C₄-alkoxycarbonyl and cyano, where the following radical combinations are excluded: dichloro, bromochloro, dibromo, trichloro, dichlorotrifluoromethyl, chlorotrifluoromethyl, bromotrifluoromethyl, chlorocyano, bromocyano, dicyano, bromomethoxy, chloromethoxy, chloroacetoxy and dimethoxy and
Het is additionally 4-pyrimidinyl, where the heterocyclic ring system carries two or three of the following radicals: fluorine, bromine, C₁-C₈-alkyl, C₃-C₆-cycloalkyl, C₁-C₂-haloalkyl, C₁-C₄-alkoxy, C₁-C₄-alkylthio, C₁-C₄-alkoxy-C₁-C₄-alkyl, aryl, aryl-C₁-C₄-alkyl, C₁-C₄-alkylcarbonyl, C₁-C₄-alkoxycarbonyl and cyano, where
the radical combinations: difluoro, trifluoro, dibromo, fluorobromo, fluorotrifluoromethyl, fluorocyano, fluoromethyl, fluoromethoxy, fluorodimethyl, bromocyano, dicyano, bromomethoxy, dimethyl, dimethoxy and methylmethoxy are excluded and
Het is additionally 5-pyrimidinyl where the heterocyclic ring system is substituted by chlorine, fluorine, C₃-C₆-cycloalkyl, C₁-C₂-haloalkyl, C₂-C₄-alkoxy, C₁-C₄-alkylthio, C₁-C₄-alkoxy-C₁-C₄-alkyl, aryl, aryl-C₁-C₄-alkyl, C₁-C₄-alkylcarbonyl, C₁-C₄-alkoxycarbonyl or cyano,
and its plant-tolerated acid addition salts and metal complexes, and the N-oxides of the heterocyclic compounds.

2. A process for the preparation of an α-arylacrylate substituted by a heterocyclic radical, of the formula I, as claimed in claim 1, wherein a heterocyclic compound of the general formula II Het-(A)ₙ-H is reacted with an α-(2-bromomethylphenyl)-acrylate of the general formula III 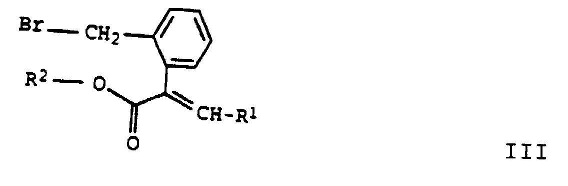 in a diluent in the presence of a base with or without a catalyst.

3. A method for controlling fungi, wherein the fungi or the materials, plants, seed or soil threatened by fungal attack are treated with a fungicidally effective amount of an α-arylacrylate substituted by a heterocyclic radical, of the general formula I, as claimed in claim 1, or with a plant-tolerated acid addition salt or metal complex thereof or with the N-oxide of the heterocyclic compound.

4. A fungicide containing an inert carrier and a fungicidally effective amount of an α-arylacrylate substituted by a heterocyclic radical, of the general formula I, as claimed in claim 1, or a plant-tolerated acid addition salt or metal complex thereof or the N-oxide of the heterocyclic compound.

5. A compound of the formula I as claimed in claim 1, wherein R¹ is methoxy, R² is methyl and Het is 2-methyl-4-quinolyl.

## Revendications

1. α-aryl-acrylester substitué hétérocycliquement de formule générale I 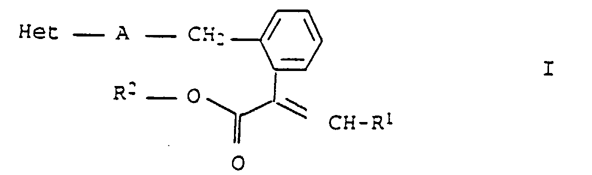 dans laquelle
R¹ représente alcoxy en C1-C4,
R² représente alkyle en C1-C4,
A est mis pour oxygène et
Het signifie 2-pyridone-1-yle, 4-pyridone-1-yle, 3-quinolyle, 4-quinolyle, 8-quinolyle, 2-pyrimidinone-1-yle, 4-pyrimidinone-1-yle ou 6-pyrimidinone-1-yle, le système annulaire hétérocyclique étant éventuellement substitué par halogène, alkyle en C1-C8, cycloalkyle en C3-C6, halogènalkyle en C1-C2, alcoxy en C1-C4, alkylthio en C1-C4, alcoxy en C1-C4-alkyle en C1-C4, aryle, aryl-alkyle en C1-C4, alkyle en C1-C4-carbonyle, alcoxy en C1-C4-carbonyle, cyano et
Het représente en outre 2-quinolinyle, le système annulaire hétérocyclique étant substitué par halogène, alkyle en C1-C8, cycloalkyle en C3-C6, halogènalkyle en C1-C2, alcoxy en C1-C4, alkylthio en C1-C4, alcoxy en C1-C4-alkyle en C1-C4, aryle, aryl-alkyle en C1-C4, alkyle en C1-C4-carbonyle, alcoxy en C1-C4-carbonyle, cyano et
Het représente, en outre, 2-pyrimidinyle, le système annulaire hétérocyclique étant monosubstitué par brome, cycloalkyle en C3-C6, halogènalkyle en C2, alcoxy en C2-C4, alkylthio en C1-C4, alcoxy en C1-C4-alkyle en C1-C4, aryl-alkyle en C1-C4, alkyle en C1-C4-carbonyle, alcoxy en C2-C4-carbonyle et
deux ou trois des restes suivants : chlore, brome, cycloalkyle en C3-C6, halogénalkyle en C1-C2, alcoxy en C1-C4, alkylthio en C1-C4, alcoxy en C1-C4-alkyle en C1-C4, aryle, aryle en C1-C4-alkyle, alkylcarbonyle en C1-C4, alcoxy en C1-C4-carbonyle et cyano, à l'exclusion des combinaisons suivantes de restes : dichlore, bromo-chlore, dibrome, trichlore, dichlorotrifluorométhyle, chloro-trifluorométhyle, bromo-trifluorométhyle, chloro-cyano, bromo-cyano, dicyano, bromo-méthoxy, chloro-méthoxy, chloro-acétoxy et diméthoxy, et
Het représente, en outre 4-pyrimidinyle dans lequel le système cyclique hétérocyclique porte deux ou trois des restes suivants : fluor, brome, alkyle en C1-C8, cycloalkyle en C3-C6, halogénalkyle en C1-C2, alcoxy en C1-C4, alkylthio en C1-C4, alcoxy en C1-C4-alkyle en C1-C4, aryle, aryle en C1-C4-alkyle, alkylcarbonyle en C1-C4, alkylcarbonyle en C1-C4 et cyano,
à l'exclusion des combinaisons de restes : difluor, trifluore, dibrome, fluoro-brome, fluoro-trifluorométhyle, fluoro-cyano, fluoro-méthyle, fluoro-méthoxy, fluoro-diméthyle, bromo-cyano, dicyano, bromo-méthoxy, diméthyle, diméthoxy et méthyl-méthoxy, et
Het représente, en outre, 5-pyrimidinyle, le système annulaire hétérocyclique étant substitué par chlore, fluor, cycloalkyle en C3-C6, halogènalkyle en C1-C2, alcoxy en C2-C4, alkylthio en C1-C4, alcoxy en C1-C4-alkyle en C1-C4, aryle, aryl-alkyle en C1-C4, alkyle en C1-C4-carbonyle, alcoxy en C1-C4-carbonyle, cyano,
ainsi que ses sels d'addition d'acide acceptables par les plantes et composés complexes métalliques et les N-oxydes des composés hétérocycliques.

2. Procédé pour la préparation d'α-arylacrylesters substitués hétérocycliquement de formule I selon la revendication 1, caractérisé par le fait que l'on fait réagir un composé hétérocyclique de formule générale II Het-(A)ₙ-H avec un α-(2-bromométhylphényl)-acrylester de la formule générale III 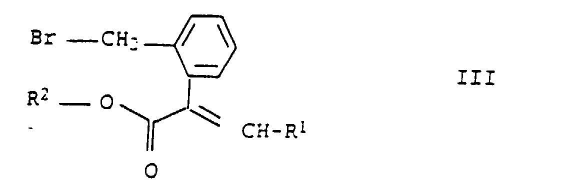 dans un diluant en présence d'une base et éventuellement en présence d'un catalyseur.

3. Procédé de lutte contre les champignons, caractérisé par le fait que l'on traite les champignons ou les matériaux, plantes, semences ou sols menacés d'une attaque par champignons, avec une quantité efficace comme fongicide d'un α-aryl-acrylester substitué hétérocycliquement de formule générale I selon la revendication 1 ou avec son sel d'addition d'acide acceptable par les plantes ou composés complexes métalliques ou les N-oxydes des composés hétérocycliques.

4. Foncigide contenant un support inerte et une quantité efficace comme fongicide d'un α-aryl-acrylester substitué hétérocycliquement de formule générale I selon la revendication 1 ou avec son sel d'addition d'acide acceptable par les plantes ou composés complexes métalliques ou les N-oxydes des composés hétérocycliques.

5. Composé de formule I selon la revendication 1, caractérisé par le fait que R¹ signifie méthoxy, R² méthyle et HET 2-méthyl-4-quinolyle.
